# EUROPEAN PATENT SPECIFICATION

(11) **EP 2 342 195 B1**
(45) Date of publication and mention of the grant of the patent: **10.09.2014**
(21) Application number: 09786058.9
(22) Date of filing: 24.07.2009
(51) Int. Cl.: C07D 403/06, A61K 31/404, A61P 35/00

(54) **CRYSTALLINE FORMS OF A 3-PYRROLE SUBSTITUTED 2-INDOLINONE MALATE SALT**
KRISTALLINE FORMEN EINES 3-PYRROLSUBSTITUIERTEN 2-INDOLINONMALATSALZES
FORMES CRISTALLINES D UN SEL DE MALATE DE 2-INDOLINONE À SUBSTITUTION 3-PYRROLE

(30) Priority: 24.07.2008 US 83330 P
(43) Date of publication of application: 13.07.2011
(73) Proprietor: Medichem, S.A., 08970 Barcelona (ES)
(72) Inventor: WINTER, Stephen, Benedict, David, E-08690 Barcelona (ES); BENITO VELEZ, Monica, E-08902 L'Hospitalet de Llobregat (EC)
(74) Representative: ABG Patentes, S.L.
(86) International application number: PCT/IB2009/006347
(87) International publication number: WO 2010/010454

(56) References cited:
- US-A1- 2007 191 458
- CAIRA M R: "CRYSTALLINE POLYMORPHISM OF ORGANIC COMPOUNDS" TOPICS IN CURRENT CHEMISTRY, SPRINGER, BERLIN, DE, vol. 198, 1 January 1998 (1998-01-01), pages 163-208, XP001156954

## Description

### BACKGROUND OF THE INVENTION

Sunitinib (compound I) is the international commonly accepted name for (Z)-*N-*[2-(diethylamino)ethyl]-5-[(*Z*)-(5-fluoro-1,2-dihydro-2-oxo-3*H*-indol-3-yliden)methyl]-2,4-dimethyl-1H-pyrrole-3-carboxamide, and has an empirical formula of C₂₂H₂₇N₄O₂F, and a molecular weight of 398.47 g/mol. Sunitinib is an active pharmaceutical substance indicated for the treatment of abnormal cell growth, such as cancer, in mammals, particularly in humans.

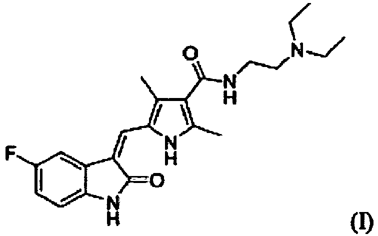

The malic acid salt of sunitinib has been selected for medical purpose and is commercially marketed under the trade name of SUTENT™ for the treatment of renal cell carcinoma and gastrointestinal stromal tumor.

Sunitinib base and its malate salt are described in U.S. Patent No. 6,573,293 ("the '293 patent"). In particular, Example 80 of the '293 patent discloses the preparation of sunitinib base.

U.S. Patent Application Publication No. 2007/0191458A1 ("the '458 publication"), discloses a number of preparations of sunitinib malate. The crystalline forms of sunitinib obtained from processes disclosed in the '458 publication are characterized by powder X-ray diffraction (XRD) and thermal techniques and are referred to as crystalline Form I and Form II.

International Patent Application Publication No. WO 2009/067686 A2 ("the '686 publication") discloses polymorphs of sunitinib racemic malate (Form A and Form B), polymorphs of sunitinib hemi-L-malate (Form E and Form U), and a number of compositions containing sunitinib and either L-malic acid (Compositions: C, F to R, V-A to V-C, and V-S)
or racemic malic acid (Composition V-T), and processes for the preparation thereof.

Polymorphism is defined as the ability of a substance to exist in two or more crystalline phases that have a different arrangement and/or conformation of the molecules in the crystal lattice. Polymorphs typically differ in their physical properties such as, for example, melting point, solubility, and chemical reactivity. Thus, the particular characteristics of the respective polymorphs can appreciably influence the solubility profile of a chemical substance, such as the dissolution rate. Further, the particular characteristics of the respective polymorphs can appreciably influence pharmaceutical properties such us dissolution rate and bioavailability.

Additionally, a crystalline form of a drug compound has advantages over an amorphous form in several respects. For example, the compound can be easily purified by crystallization and recrystallization. Crystallization is a much cheaper and more convenient method of purification to perform on a large scale than certain other methods of purification such as chromatography. Further, a crystalline form is usually more stable than an amorphous form, both before and during formulation and during subsequent storage.

It would be desirable, therefore, to identify and isolate various crystalline polymorphic forms of sunitinib malate. Further, it would be desirable to have reliable processes for producing sunitinib malate in one or more of its polymorphic forms. Additionally, the various polymorphic forms of sunitinib malate could be used to prepare improved pharmaceutical compositions.

In view of the foregoing, there is an unmet need for novel crystalline polymorphic forms of sunitinib malate. Further, there is an unmet need for processes for preparing novel crystalline polymorphic forms of sunitinib malate and for pharmaceutical compositions comprising novel crystalline polymorphic forms of sunitinib malate.

### BRIEF SUMMARY OF THE INVENTION

The invention provides a crystalline polymorphic forms of the L-malic acid salt of (Z)-*N*-[2-(diethylamino)ethyl]-5-[(5-fluoro-1,2-dihydro-2-oxo-3*H*-indol-3-yliden)methyl]-2,4-dimethyl-1*H*-pyrrole-3-carboxamide, sunitinib L-malate, as defined in claim 1. The present invention also provides processes for preparing said crystalline polymorphic form of sunitinib L-malate. The present invention further provides pharmaceutical compositions comprising said crystalline polymorphic form of sunitinib L-malate.

### BRIEF DESCRIPTION OF THE DRAWINGS

Figure 1 depicts the X-ray powder diffractogram (XRD) of sunitinib L-malate Form I obtained according to the process of Reference Example 1, which is not encompassed by the present invention.
Figure 2 depicts the Infrared (IR) spectra of sunitinib L-malate Form I obtained according to the process of Reference Example 1, which is not encompassed by the present invention.
Figure 3 depicts the XRD of sunitinib L-malate Form III obtained according to the process of Example 2.
Figure 4 depicts the IR spectra of sunitinib L-malate Form III obtained according to the process of Example 2.
Figure 5 depicts the XRD of sunitinib L-malate Form IV obtained according to the process of Reference Example 3, which is not encompassed by the present invention.
Figure 6 depicts the IR spectra of sunitinib L-malate Form IV obtained according to the process of Reference Example 3, which is not encompassed by the present invention.
Figure 7 depicts the XRD of sunitinib L-malate Form V obtained according to the process of Reference Example 4, which is not encompassed by the present invention.
Figure 8 depicts the IR spectra of sunitinib L-malate Form V obtained according to the process of Reference Example 4, which is not encompassed by the present invention.
Figure 9 depicts the XRD of sunitinib L-malate Form VI obtained according to the process of Reference Example 5, which is not encompassed by the present invention.
Figure 10 depicts the IR spectra of sunitinib L-malate Form VI obtained according to the process of Reference Example 5, which is not encompassed by the present invention.
Figure 11 depicts the XRD of sunitinib L-malate Form VII obtained according to the process of Reference Example 6, which is not encompassed by the present invention.
Figure 12 depicts the IR spectra of sunitinib L-malate Form VII obtained according to the process of Reference Example 6, which is not encompassed by the present invention.
Figure 13 depicts the XRD of low crystalline sunitinib L-malate obtained after storing sunitinib L-malate Form IV under standard conditions for a period of time, which is not encompassed by the present invention.

### DETAILED DESCRIPTION OF THE INVENTION

Applicants have discovered crystalline forms of sunitinib malate, i.e., sunitinib hemimalate. These forms of sunitinib malate have been prepared and characterized and are referred to as Forms III, IV, V, VI and VII.

In addition, a selected group of the crystalline forms of sunitinib malate , i.e., Forms III and V, have been found to be highly stable in terms of polymorphic form, which makes them suitable for pharmaceutical formulation use.

Crystalline polymorphic forms of the malic acid salt of (*Z*)-*N*-[2-(diethylamino)ethyl]-5-[(5-fluoro-1,2-dihydro-2-oxo-3*H*-indol-3-yliden)methyl]-2,4-dimethyl-1H-pyrrole-3-carboxamide, sunitinib malate, crystalline Forms III, IV, V, VI and VII, are described herein. Processes for preparing the crystalline forms of sunitinib malate are also described. Further, pharmaceutical compositions comprising the crystalline forms of sunitinib malate, Forms III, IV, V, VI and VII are also described.

The malic acid of the salts of sunitinib may be D-malic acid salt; D,L-malic acid salt; L-malic acid salt; or combinations thereof. According to the present invention, the malic acid salt of sunitinib is the L-malic acid salt.

The present invention provides a polymorphic Form III of the malic acid salt of sunitinib (hereinafter referred to as sunitinib malate Form III).

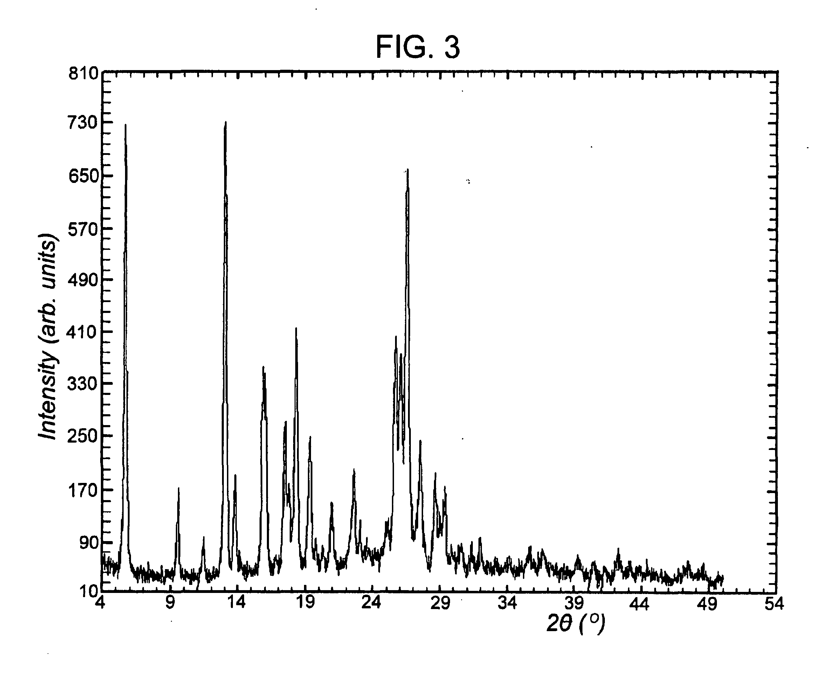

Figure 3 depicts the X-ray powder diffraction pattern (2θ) of sunitinib malate Form III, which shows characteristic peaks at approximately 5.7, 13.1, 15.9, 16.0, 17.5, 18.3, 19.3, 25.7, 26.1 and 26.6° and with further peaks at 9.6, 11.4, 13.8, 17.8, 20.9, 22.6, 27.5, 28.6 and 29.4 degrees (± 0.2°).

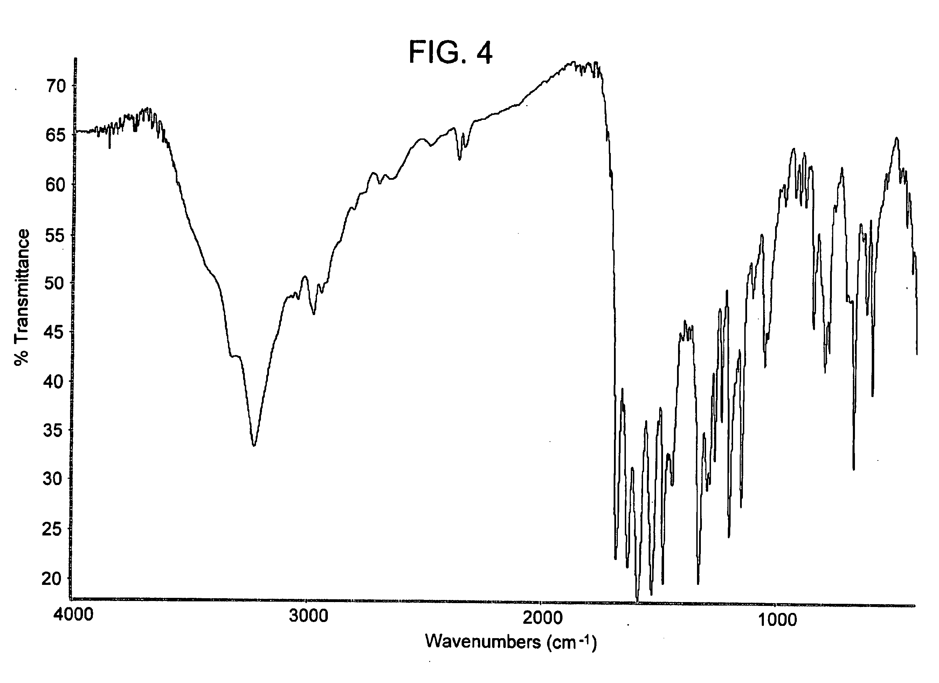

Figure 4 depicts the IR spectrum of sunitinib malate Form III, which shows characteristic absorption bands at approximately 3226.3, 2978.7, 1679.3, 1627.6, 1586.5, 1526.4, 1477.9, 1440.5, 1326.8, 1290.7, 1259.4, 1230.8, 1196.8, 1145.2, 1050.2, 841.2, 792.8, 774.1, 666.9, 614.3 and 588.3 cm⁻¹ and with further absorption bands at 1101.2, 966.4, 921.0, 901.0, 876.8, 444.1 and 418.6 cm⁻¹.

The crystalline sunitinib malate Form III of the invention has been found to be highly stable in terms of polymorphic form. For example, a sample of sunitinib malate Form III was stored under standard conditions (i.e., room temperature, normal pressure, and ambient atmosphere) for a period of time, for example, at least one year, and was retested for crystalline stability. The X-ray powder diffractogram (XRD) obtained was substantially identical to the XRD depicted in Figure 3.

A method for preparing crystalline sunitinib malate Form III, not according to the present invention, is provided which comprises i) mixing sunitinib malate with a selected solvent to form a suspension or solution, and ii) removing the solvent from said mixture to form sunitinib malate Form III.

In said method, the solvent comprises, for example, ethanol.

In said method step i) can be carried out at a temperature of between about 0 °C and about 200 °C.

In said method step ii) can be carried out, for example, by filtering the suspension or by evaporation.

According to the invention, the method for preparing crystalline sunitinib L-malate Form III of the invention comprises i) forming a solution of sunitinib L-malate in a solvent comprising ethanol, ii) optionally, precipitating the sunitinib L-malate polymorphic Form III from the solution to form a suspension, and iii) removing the solvent.

In a preferred embodiment, the solvent for preparing crystalline sunitinib malate Form III is free, or substantially free of water. For example, solvents for preparing crystalline sunitinib malate Form III comprise less than 1% water, less than 0.5% water, or less than 0.2% water.

In a particularly preferred embodiment, the solvent for preparing crystalline sunitinib malate Form III is absolute ethanol comprising not more than about 0.5% water.

In keeping with the invention, the removing the solvent of step iii) comprises filtering the suspension of step ii). In an alternative embodiment, the removing the solvent of step iii) comprises evaporating the solvent.

The sunitinib malate used for preparing sunitinib malate Form III can be either sunitinib malate obtained by any suitable method, or sunitinib malate crystalline forms I, II, IV, V, VI, VII, or mixtures thereof.

A polymorphic Form IV of the malic acid salt of sunitinib (hereinafter referred to as sunitinib malate Form IV) , which is not encompassed by the present invention, is described herein.

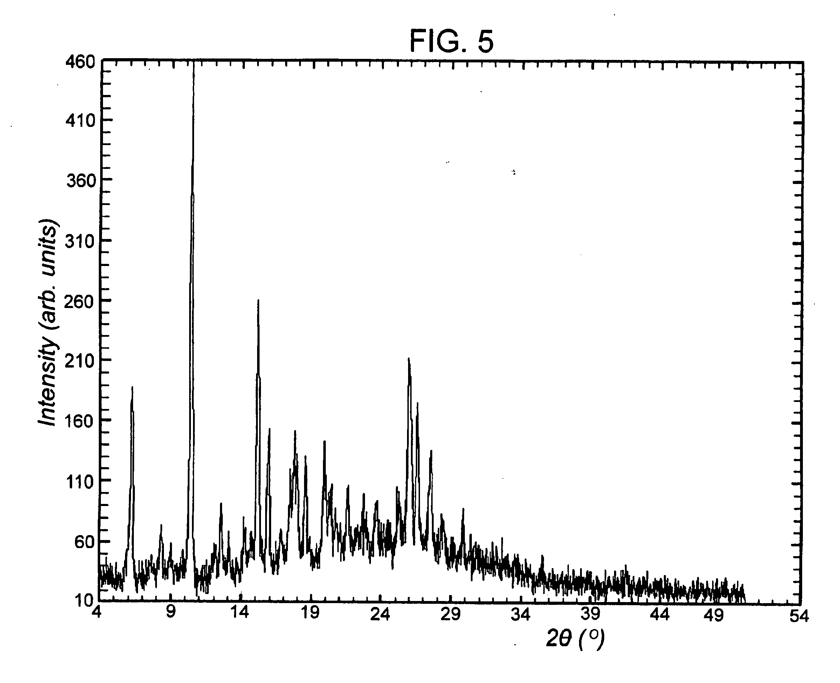

Figure 5 depicts the X-ray powder diffraction pattern (2θ) of sunitinib malate Form IV, which is not encompassed by the present invention, which shows characteristic peaks at approximately 6.2, 10.4, 15.1, 15.9, 17.7, 18.6, 26.0, 26.5 and 27.5° and with further peaks at 8.3, 12.5, 19.9, 21.6, 23.6 and 25.2 degrees (± 0.2°).

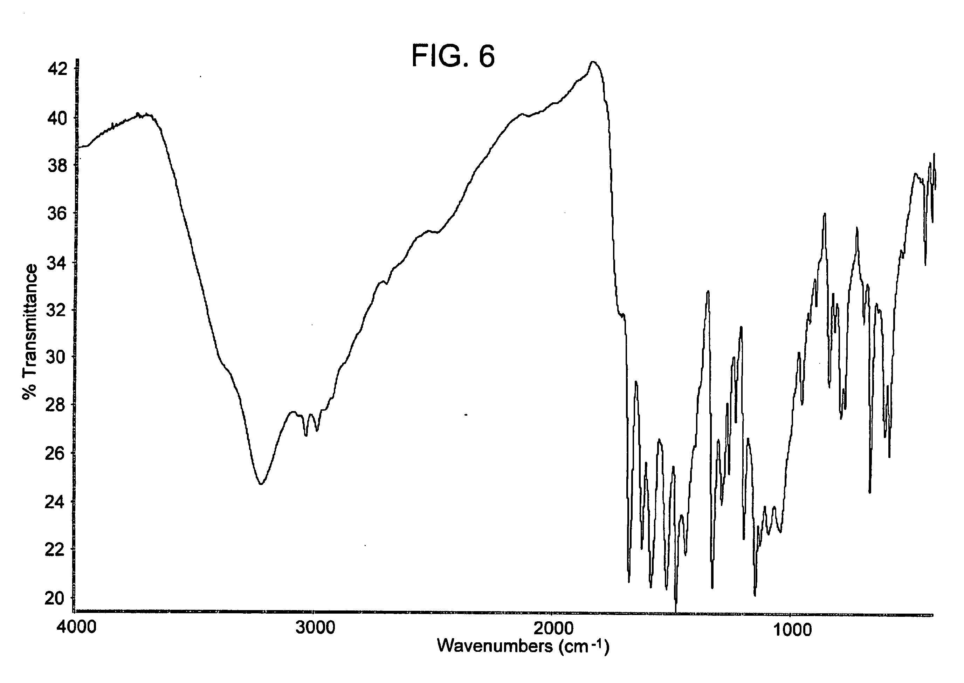

Figure 6 depicts the IR spectrum of sunitinib malate Form IV, which is not encompassed by the present invention, which shows characteristic absorption bands at approximately 3220.2, 3034.9, 1677.6, 1622.8, 1586.3, 1519.8, 1479.0, 1440.6, 1327.1, 1288.1, 1259.9, 1231.9, 1195.8, 1146.3, 1127.6, 1093.6, 1043.2, 956.1, 841.1, 792.2, 775.4, 667.1, 609.5 and 588.1 cm⁻¹ and with further absorption bands at 897.8, 819.7, 697.8 and 443.1 cm⁻¹.

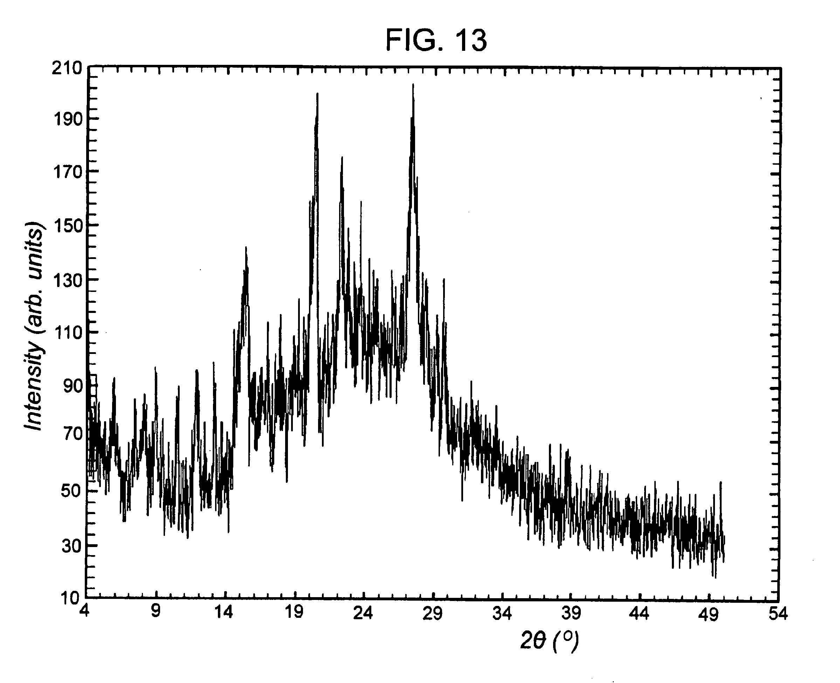

The sunitinib malate Form IV has been found to lose its crystallinity with the passage of time. For example, a sample of sunitinib malate Form IV was stored under standard conditions (i.e., room temperature, normal pressure, and ambient atmosphere) for a period of time, for example, at least one year and was retested for crystalline stability. The X-ray powder diffractogram (XRD) obtained is depicted in Figure 13.

Methods for preparing sunitinib malate Form IV are described, comprising i) mixing sunitinib malate with a selected solvent to form a suspension or solution, and ii) removing the solvent from said mixture to form sunitinib malate Form IV.

Preferably, the solvent comprises, for example, dimethyl sulfoxide (DMSO).

Step i) can be carried out at a temperature of between about 0 °C and about 200 °C.

Step ii) can be carried out, for example, by filtering the suspension or by evaporation.

The sunitinib malate used for preparing sunitinib malate Form IV can be either sunitinib malate obtained by any suitable method, or sunitinib malate crystalline forms I, II, III, V, VI, VII, or mixtures thereof.

A polymorphic Form V of the malic acid salt of sunitinib (hereinafter referred to as sunitinib malate Form V), which is not encompassed by the present invention, is described herein.

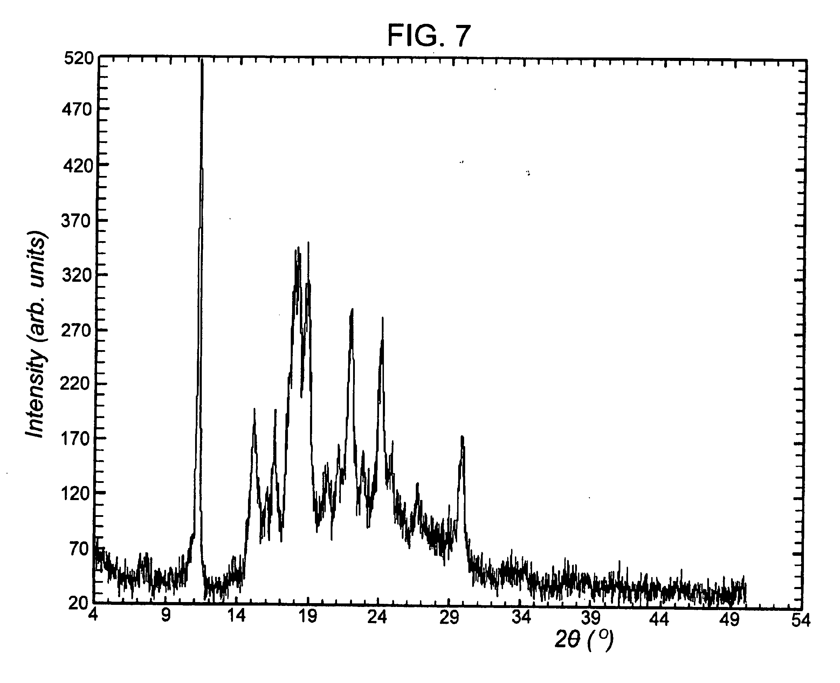

Figure 7 depicts the X-ray powder diffraction pattern (2θ) of sunitinib malate Form V, which is not encompassed by the present invention, which shows characteristic peaks at approximately 11.2, 15.1, 16.5, 17.9, 18.2, 18.8, 21.9, 24.0 and 29.8 degrees (± 0.2°).

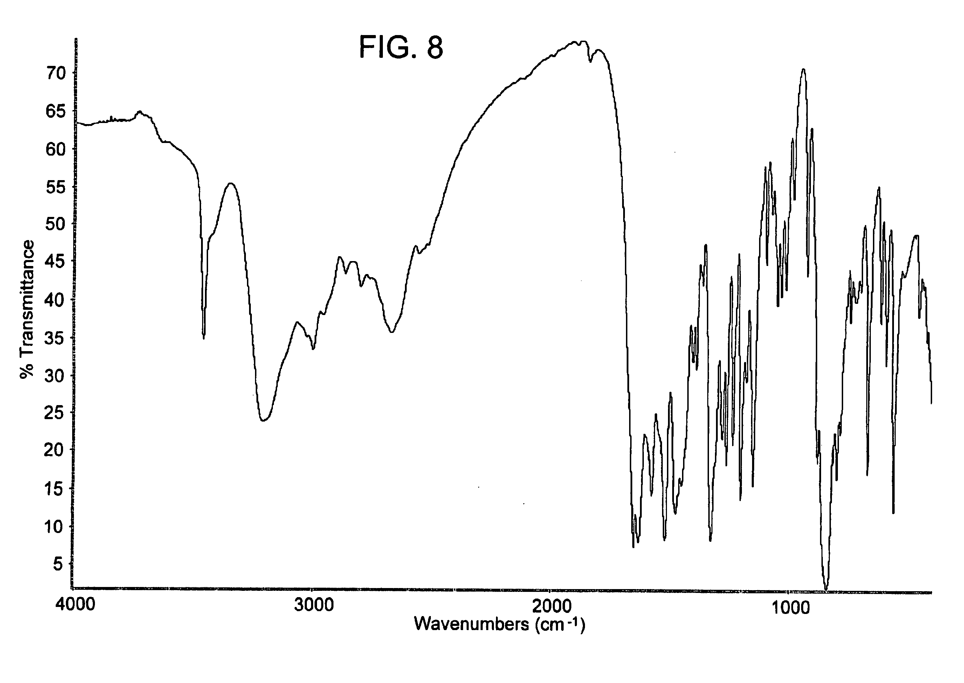

Figure 8 depicts the IR spectrum of sunitinib malate Form V, which is not encompassed by the present invention, which shows characteristic absorption bands at approximately 3459.6, 3207.1, 2999.3, 2672.3, 1649.0, 1627.3, 1574.1, 1519.5, 1473.5, 1402.7, 1388.1, 1326.3, 1277.4, 1261.1, 1234.4, 1201.0, 1177.9, 1148.1, 1048.9, 1034.5, 875.6, 840.3, 794.4, 738.1, 717.3, 664.8, 612.1, 588.6, 557.4 and 452.4 cm⁻¹ and with further absorption bands at 1361.5, 1095.8, 1071.5, 1012.8, 982.1 and 923.4 cm⁻¹.

The sunitinib malate Form V has been found to be highly stable in terms of polymorphic form. For example, a sample ofsunitinib malate Form V was stored under standard conditions (i.e., room temperature, normal pressure, and ambient atmosphere) for a period of time, for example, at least one year and was retested for crystalline stability.
The X-ray powder diffractogram (XRD) obtained was substantially identical to the XRD
depicted in Figure 7.

A method for preparing sunitinib malate Form V is described, comprising i) forming a solution or suspension of sunitinib malate in a selected solvent, and ii) removing the solvent from said mixture to form sunitinib malate Form V.

Preferably, the solvent comprises, for example, a mixture of butanol and water.

Step i) can be carried out at a temperature of between about 0 °C and about 200 °C.

Step ii) can be carried out, for example, by filtering the suspension or by evaporation.

Preferably, the method for preparing sunitinib malate Form V comprises i) forming a solution of sunitinib malate in a solvent comprising at least one C₁-C₅ alcohol and water, ii) optionally, precipitating the sunitinib malate polymorphic Form V to form a suspension, and iii) removing the solvent.

The solvent comprising a mixture of at least one C₁-C₅ alcohol and water preferably comprises a mixture of at least one C₁-C₅ alcohol solvent with up to 50% (v/v) of water, more preferably, the solvent comprising a mixture of at least one C₁-C₅ alcohol and water comprises a mixture of at least one C₁-C₅ alcohol solvent with 20% (v/v) of water.

Preferably, the at least one C₁-C₅ alcohol is butanol.

Removing the solvent of step iii) comprises filtering the suspension of step ii). Alternatively, removing the solvent of step iii) comprises evaporating the solvent.

The sunitinib malate used for preparing sunitinib malate Form V can be either sunitinib malate obtained by any suitable method, or sunitinib malate crystalline forms I, II, III, IV, VI, VII, or mixtures thereof.

A polymorphic Form VI of the malic acid salt of sunitinib (hereinafter referred to as sunitinib malate Form VI), which is not encompassed by the present invention, is described herein.

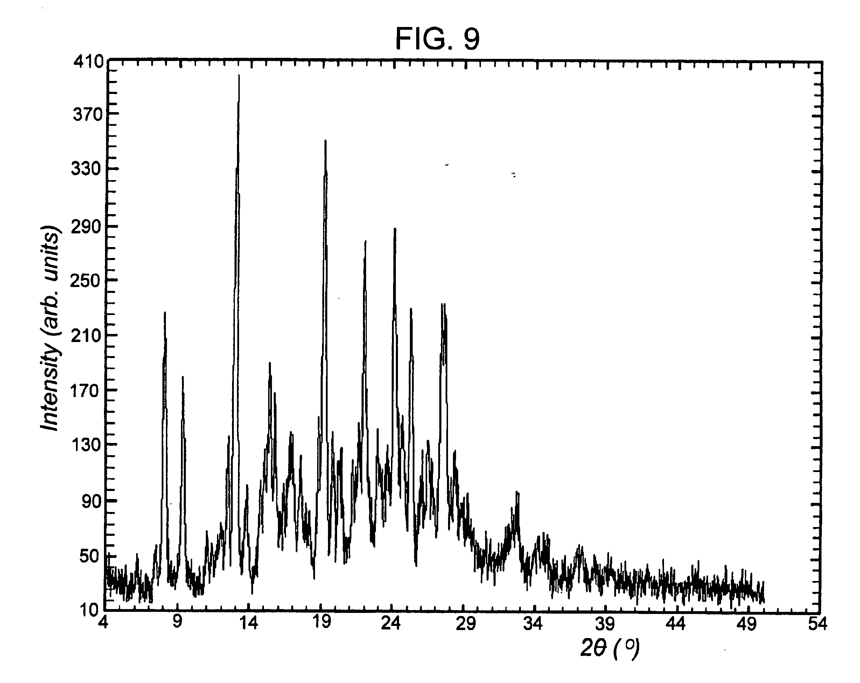

Figure 9 depicts the X-ray powder diffraction pattern (2θ) of sunitinib malate Form VI, which is not encompassed by the present invention, which shows characteristic peaks at approximately 8.1, 9.3, 13.0, 15.4, 19.2, 22.0, 24.1, 25.3, 27.4 and 27.7° and with further peaks at 12.5, 13.8, 15.8, 16.9, 18.8, 19.7, 20.2, 21.6 and 24.6 degrees (± 0.2°).

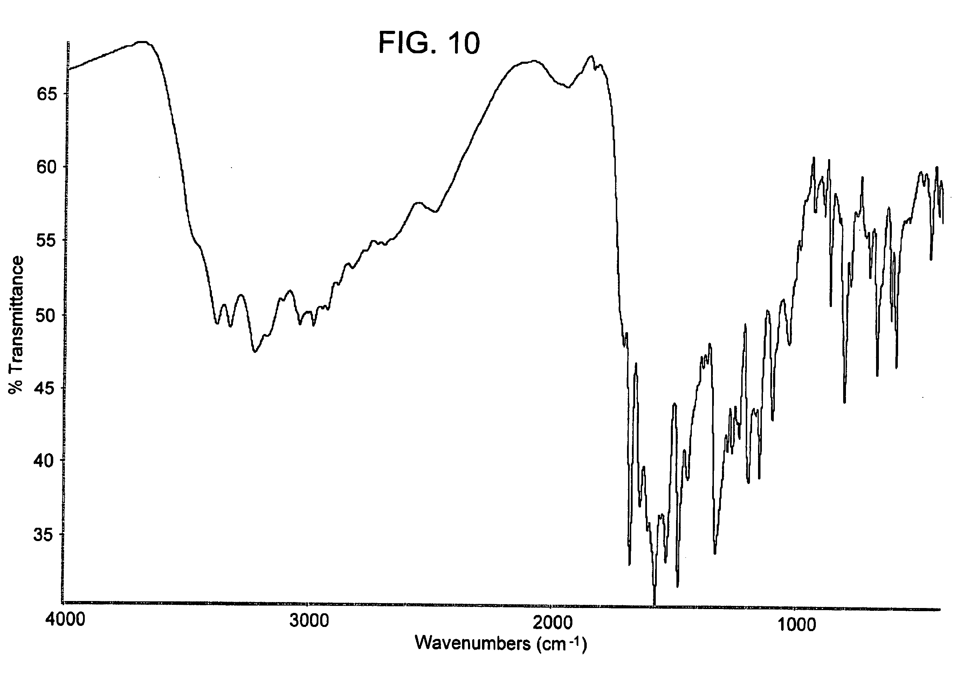

Figure 10 depicts the IR spectrum of sunitinib malate Form VI, which is not encompassed by the present invention, which shows characteristic absorption bands at approximately 3327.7, 3225.2, 2984.6, 1677.0, 1634.7, 1572.6, 1528.5, 1478.6, 1441.2, 1326.8, 1260.6, 1230.5, 1192.4, 1146.9, 1096.1, 1028.2, 797.9, 663.6, 606.5 and 585.4 cm⁻¹ and with further absorption bands at 925.1, 883.2, 858.5, 773.8, 695.4, 445.4 and 413.4 cm⁻¹.

The sunitinib malate Form VI has been found to be a mixture of sunitinib malate known Form I and at least one unknown crystalline form.

Further, a sample of sunitinib malate Form VI (i.e., mixture of sunitinib malate known Form I and at least one unknown crystalline form) was stored under standard conditions (i.e., room temperature, normal pressure, and ambient atmosphere) for a period of time, for example, at least one year and was retested for crystalline stability. The X-ray powder diffractogram (XRD) obtained showed a clear conversion of the unknown crystalline form to known Form I.

Methods for preparing sunitinib malate Form VI are also described, comprising i) mixing sunitinib malate with a selected solvent, to obtain a suspension or solution, and ii) removing the solvent from said mixture, to give sunitinib malate Form VI.

Preferably, the solvent comprises, for example, nitromethane, *N,N-*dimethylformamide (DMF), and mixtures thereof.

Step i) can be carried out at a temperature of between about 0 °C and about 200 °C.

Step ii) can be carried out, for example, by filtering the suspension or by evaporation.

The sunitinib malate used for preparing sunitinib malate Form VI can be either sunitinib malate obtained by any suitable method, or sunitinib malate crystalline forms I, II, III, IV, V, VII, or mixtures thereof.

A polymorphic Form VII of the malic acid salt of sunitinib (hereinafter referred to as sunitinib malate Form VII), which is not encompassed by the present invention, is described.

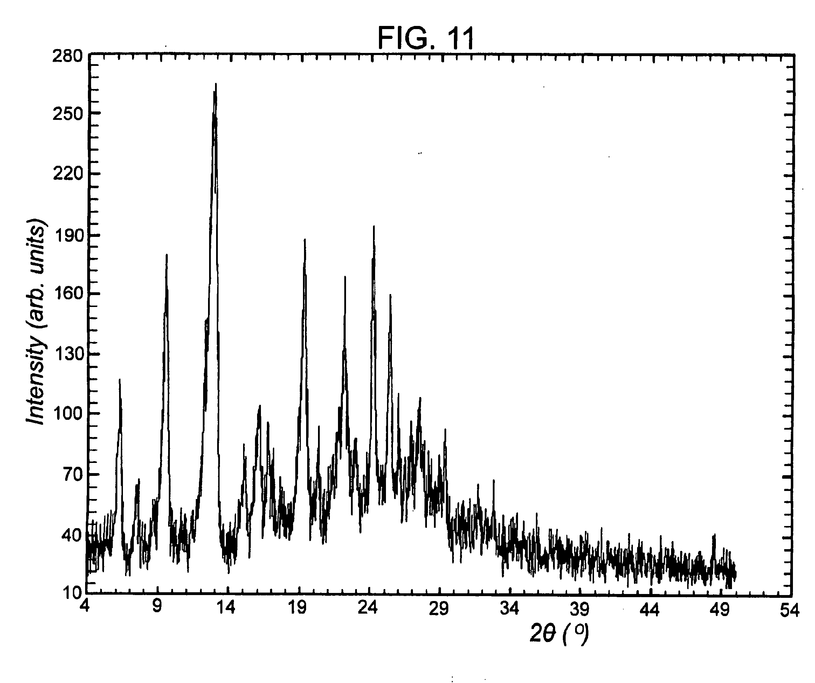

Figure 11 depcits the X-ray powder diffraction pattern (2θ) of sunitinib malate Form VII, which is not encompassed by the present invention, which shows characteristic peaks at approximately 6.1, 9.4, 12.8, 16.1, 19.2, 22.1, 24.1 and 25.3° and with further peaks at 7.4, 15.1, 16.7, 40.5 degrees (± 0.2°).

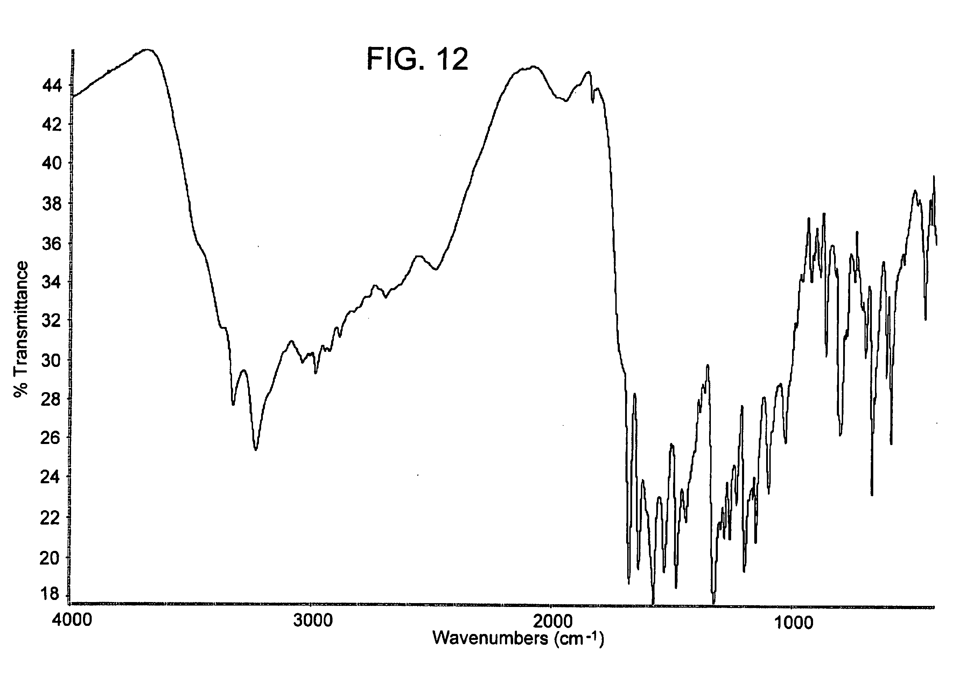

Figure 12 depicts the IR spectrum of sunitinib malate Form VII, which is not encompassed by the present invention, which shows characteristic absorption bands at approximately 3326.8, 3229.6, 2984.4, 1674.0, 1634.1, 1572.9, 1528.2, 1477.5, 1439.6, 1322.4, 1277.5, 1255.8, 1229.4, 1194.5, 1146.6, 1095.5, 1027.2, 859.6, 797.6, 694.1, 663.3, 605.1, 585.3 and 446.4 cm⁻¹ and with further absorption bands at 921.6, 883.6, 737.6 and 418.5 cm⁻¹.

The sunitinib malate Form VII has been found to be a mixture of sunitinib malate known Form I and at least one unknown crystalline form.

Further, a sample of sunitinib malate Form VII (i.e., mixture of sunitinib malate known Form I and at least one unknown crystalline form) was stored under standard conditions (i.e., room temperature, normal pressure, and ambient atmosphere) for a period of time, for example, at least one year and was retested for crystalline stability. The X-ray powder diffractogram (XRD) obtained showed a clear conversion of the unknown crystalline form to known Form I.

Methods for preparing sunitinib malate Form VII are also described, comprising i) mixing sunitinib malate with a selected solvent, to form a suspension or solution, and ii) removing the solvent from said mixture, to form sunitinib malate Form VII.

Preferably, the solvent comprises, for example, 2,2,2-trifluoroethanol.

Step i) can be carried out at a temperature of between about 0 °C and about 200 °C.

Step ii) can be carried out, for example, by filtering the suspension or by evaporation.

The sunitinib malate used for preparing sunitinib malate Form VII can be either sunitinib malate obtained by any suitable method or sunitinib malate crystalline forms I, II, III, IV, V, VI, or mixtures thereof.

Pharmaceutical compositions comprising sunitinib malate in any of crystalline Forms III, IV, V, VI and VII, or mixtures thereof are also described. In one embodiment of the invention the pharmaceutical composition of the invention comprises sunitinib malate crystalline Form III.

In keeping with embodiments of the invention, pharmaceutical compositions of the invention comprise a pharmaceutical carrier, as appropriate.

The following examples further illustrate the invention but, of course, should not be construed as in any way limiting its scope.

XRD diffractograms were obtained using a RX SIEMENS D5000 diffractometer with a vertical goniometer, a copper anodic tube, and radiation CuK_{α}, λ=1.54056 A.

Fourier transform IR spectra were acquired on a Thermo Nicolet Nexus spectrometer. Polymorphs were characterized in potassium bromide pellets.

### Reference Example 1: Preparation of sunitinib malate Form I (not encompassed by the present invention).

This example illustrates a process for preparing sunitinib L-malate Form I.

Sunitinib base (13 g) was suspended in 2 L of methanol at ambient temperature and L-malic acid (4.7 g) was added. The mixture was stirred for 30 minutes and then concentrated under vacuum to dryness. Acetonitrile (500 mL) was added and the mixture was stirred at 70 °C for 1 hour. After cooling to ambient temperature the solid was collected by filtration and dried under vacuum at 40 °C for 48 hours.

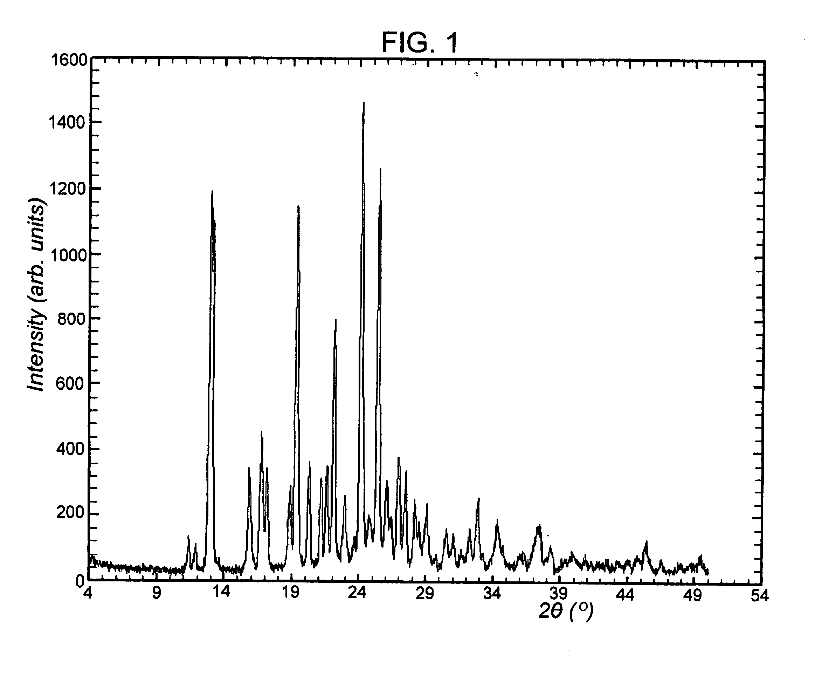

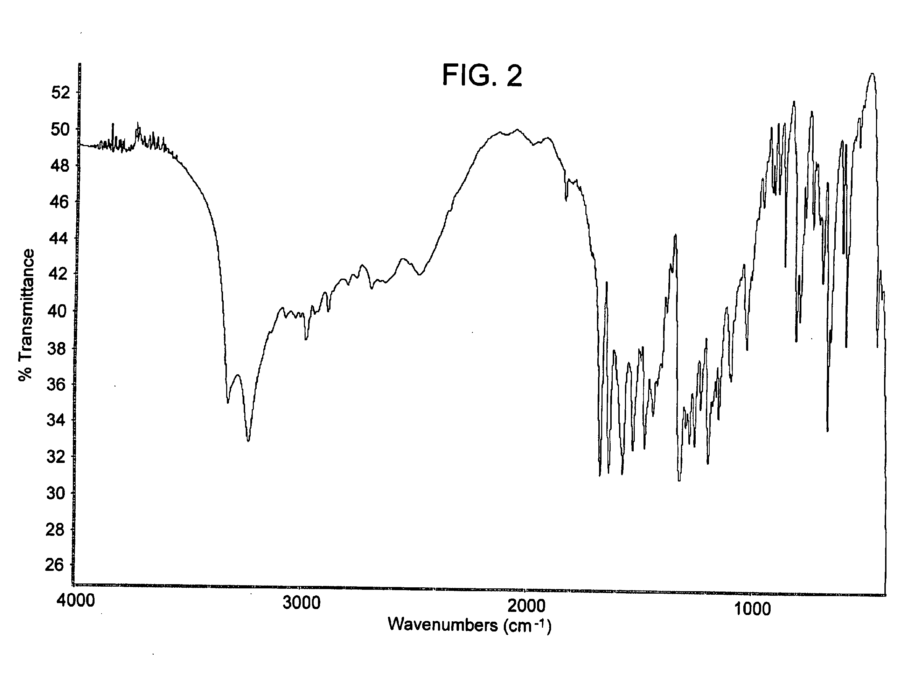

Analytical data: XRD: Form I, as shown in Figure 1; IR as shown in Figure 2.

### Example 2: Preparation of sunitinib malate Form III.

This example illustrates a process for preparing sunitinib L-malate Form III in accordance with an embodiment of the invention.

Sunitinib malate (100 mg) was dissolved in ethanol (20 mL) at reflux. After cooling to ambient temperature the solid was collected by filtration and dried under vacuum at ambient temperature.

Analytical data: XRD: Form III, as shown in Figure 3; IR as shown in Figure 4. Sunitinib L-malate crystalline Form III has the XRD shown in Figure 3 and the IR shown in Figure 4.

### Reference Example 3: Preparation of sunitinib malate Form IV (not encompassed by the present invention).

This example illustrates a process for preparing sunitinib L-malate Form IV in accordance with an embodiment of the invention.

Sunitinib malate (100 mg) was dissolved in DMSO (1 mL) with heating. After cooling to ambient temperature the mixture was stirred for 24 hours and then concentrated under vaccum at 40 °C for 4 weeks.

Analytical data: XRD: Form IV, as shown in Figure 5; IR as shown in Figure 6. Sunitinib L-malate crystalline Form IV has the XRD shown in Figure 5 and the IR shown in Figure 6.

### Reference Example 4: Preparation of sunitinib malate Form V (not encompassed by the present invention).

This example illustrates a process for preparing sunitinib L-malate Form V in accordance with an embodiment of the invention.

Sunitinib base (130 mg) was dissolved in 80:20 butanol:water (15 mL) at 80 °C. L-Malic acid (47 mg) was added and after 10 minutes at 80 °C the mixture was cooled to ambient temperature. The solid was filtered and dried under vacuum at 40 °C for 72 hours.

Analytical data: XRD: Form V, as shown in Figure 7; IR as shown in Figure 8. Sunitinib L-malate crystalline Form V has the XRD shown in Figure 7 and the IR shown in Figure 8.

### Reference Example 5: Preparation of sunitinib malate Form VI (not encompassed by the present invention).

This example illustrates a process for preparing sunitinib L-malate Form VI in accordance with an embodiment of the invention.

Sunitinib malate (100 mg) was dissolved in nitromethane (10 mL) with heating.
After cooling to ambient temperature the mixture was stirred for 24 hours and the solvent evaporated.

Analytical data: XRD: Form VI, as shown in Figure 9 (i.e., mixture of sunitinib malate known Form I and at least one unknown crystalline form). IR as shown in Figure 10.
Sunitinib L-malate crystalline Form VI has the XRD shown in Figure 9 and the IR shown in Figure 10.

### Reference Example 6: Preparation of sunitinib malate Form VII (not encompassed by the present invention).

This example illustrates a process for preparing sunitinib L-malate Form VII in accordance with an embodiment of the invention.

Sunitinib malate (100 mg) was dissolved in 2,2,2-trifluoroethanol (1 mL) with heating. After cooling to ambient temperature the mixture was stirred for 24 hours and then concentrated under vacuum at 40 °C for 2 weeks.

Analytical data: XRD: Form VII, as shown in Figure 11 (i.e., mixture of sunitinib malate known Form I and at least one unknown crystalline form); IR as shown in Figure 12.
Sunitinib L-malate crystalline Form VII has the XRD shown in Figure 11 and the IR shown in Figure 12.

### Reference Examples 7-9: Other preparations of sunitinib malate polymorphs (not encompassed by the present invention).

This example illustrates a process for preparing sunitinib L-malate Form VI in accordance with an embodiment of the invention.

Sunitinib malate (100 mg) was dissolved with heating in the solvent indicated in the table below. After cooling to ambient temperature the mixture was stirred for 24 hours and then concentrated under vacuum at 40 °C.

**Table 1**

| **Example** | **Solvent** | **Volume** | **XRD Result** |
|---|---|---|---|
| **7** | DMF | 1 | Form VI |
| **8** | 2-ethoxyethanol | 1 | Form I |
| **9** | DMA | 1.5 | Form I |

Analytical data of Example 7: XRD: Form VI, as shown in Figure 9 (i.e., mixture of sunitinib malate known Form I and at least one unknown crystalline form). Sunitinib L-malate crystalline Form VI has the XRD shown in Figure 9 and the IR shown in Figure 10.

Analytical data of Examples 8 and 9: XRD: Form I, as shown in Figure 1; Sunitinib L-malate crystalline Form I has the XRD shown in Figure 1 and the IR shown in Figure 2.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law.

## Claims

1. Sunitinib L-malate polymorphic Form III having an X-ray powder diffraction pattern (2θ) comprising characteristic peaks at approximately 5.7, 13.1, 15.9, 16.0, 17.5, 18.3, 19.3, 25.7, 26.1 and 26.6 degrees.

2. The sunitinib L-malate polymorphic Form III of claim 1, having an X-ray powder diffraction pattern (2θ) further comprising characteristic peaks at approximately 9.6, 11.4, 13.8, 17.8, 20.9, 22.6, 27.5, 28.6 and 29.4 degrees.

3. A process for preparing the sunitinib L-malate polymorphic Form III of claim 1 or claim 2, said process comprising:
i) forming a solution of sunitinib L-malate in a solvent comprising ethanol;
ii) optionally, precipitating the sunitinib L-malate polymorphic Form III from the solution to form a suspension; and
iii) removing the solvent.

4. The process of claim 3, wherein removing the solvent comprises filtering the suspension of step ii).

5. The process of claim 3, wherein removing the solvent comprises evaporating the solvent.

6. A pharmaceutical composition comprising the sunitinib L-malate polymorphic form of any one of claims 1 or 2.

## Patentansprüche

1. Polymorphe Form III von Sunitinib L-Malat mit einem Röntgenpulver-Diffraktionsmuster (2θ), umfassend charakteristische Maxima bei näherungsweise 5.7, 13.1, 15.9, 16.0, 17.5, 18.3, 19.3, 25.7, 26.1 und 26.6 Grad.

2. Polymorphe Form III von Sunitinib L-Malat nach Anspruch 1 mit einem Röntgenpulver-Diffraktionsmuster (2θ), weiterhin umfassend charakteristische Maxima bei näherungsweise 9.6, 11.4, 13.8, 17.8, 20.9, 22.6, 27.5, 28.6 und 29.4 Grad.

3. Verfahren zum Herstellen der polymorphen Form III von Sunitinib L-Malat nach Anspruch 1 oder Anspruch 2, wobei das Verfahren umfasst:
i) Bilden einer Lösung von Sunitinib L-Malat in einem Lösungsmittel, umfassend Ethanol;
ii) fakultativ Ausfällen der polymorphen Form III von Sunitinib L-Malat aus der Lösung, um eine Suspension zu bilden; und
iii) Entfernen des Lösungsmittels.

4. Verfahren nach Anspruch 3, wobei das Entfernen des Lösungsmittels das Filtern der Suspension von Schritt ii) umfasst.

5. Verfahren nach Anspruch 3, wobei das Entfernen des Lösungsmittels das Verdampfen des Lösungsmittels umfasst.

6. Pharmazeutische Zusammensetzung, umfassend die polymorphe Form von Sunitinib L-Malat nach einem der Ansprüche 1 oder 2.

## Revendications

1. Forme polymorphe III de L-malate de sunitinib possédant un diagramme de diffraction des rayons X sur poudre (2θ) comprenant des pics caractéristiques à environ 5,7, 13,1, 15,9, 16,0, 17,5, 18,3, 19,3, 25,7, 26,1 et 26,6 degrés.

2. Forme polymorphe III de L-malate de sunitinib selon la revendication 1, possédant un diagramme de diffraction des rayons X sur poudre (2θ) comprenant en outre des pics caractéristiques à environ 9,6, 11,4, 13,8, 17,8, 20,9, 22,6, 27,5, 28,6, et 29,4 degrés.

3. Procédé de préparation de la forme polymorphe III de L-malate de sunitinib selon la revendication 1 ou la revendication 2, ledit procédé consistant à :
i) former une solution de L-malate de sunitinib dans un solvant comprenant de l'éthanol ;
ii) éventuellement, précipiter la forme polymorphe III de L-malate de sunitinib de la solution pour former une suspension; et
iii) enlever le solvant.

4. Procédé selon la revendication 3, dans lequel l'opération qui consiste à enlever le solvant comporte le filtrage de la suspension de l'étape ii).

5. Procédé selon la revendication 3, dans lequel l'opération qui consiste à enlever le solvant comporte l'évaporation du solvant.

6. Composition pharmaceutique comprenant la forme polymorphe III de L-malate de sunitinib selon la revendication 1 ou 2.
